# EUROPEAN PATENT SPECIFICATION

(11) **EP 3 655 969 B1**
(45) Date of publication and mention of the grant of the patent: **16.09.2026**
(21) Application number: 18788881.3
(22) Date of filing: 10.07.2018
(51) Int. Cl.: G16H 20/40, G16H 40/63, G16B 99/00

(54) **METHOD AND INTERACTIVE SYSTEM FOR ENHANCING PERFORMANCE AND USER EXPERIENCE WITH A PUMP**
VERFAHREN UND INTERAKTIVES SYSTEM ZUR VERBESSERUNG DER LEISTUNG UND BENUTZERERFAHRUNG MIT EINER PUMPE
PROCÉDÉ ET SYSTÈME INTERACTIF PERMETTANT D'AMÉLIORER LES PERFORMANCES ET L'EXPÉRIENCE D'UTILISATEUR AVEC UNE POMPE

(30) Priority: 18.07.2017 US 201762534196 P
(43) Date of publication of application: 27.05.2020
(73) Proprietor: Medela Holding AG, 6340 Baar (CH)
(72) Inventor: GIST, Jahi, Barrington IL 60010 (US)
(74) Representative: Grünecker Patent- und Rechtsanwälte PartG mbB
(86) International application number: PCT/IB2018/000738
(87) International publication number: WO 2019/016586

(56) References cited:
- US-A1- 2014 108 041
- US-A1- 2016 144 150
- US-A1- 2016 220 743
- US-B2- 10 220 141

## Description

### FIELD OF DISCLOSURE

The present disclosure relates generally to a method and interactive system for enhancing performance and user experience with a pump, such as a breastpump or wound therapy pump. Specifically, improved user efficiencies and feedback are realized in a system constructed in accordance with the principles herein.

### BACKGROUND

Systems that assist pump users can include various types of feedback, such as flow data or timing mechanism alarms. Other systems can collect physiological data associated with the use of a pump that can provide data to the user or pump during operation. See for example US Patent No. 8,597,234 to Michael Larsson. Such applications typically limit the feedback to a single specific parameter associated with the pump session, such as the flow of milk or fluid during the session, or physical change of the user.

However, known systems are not designed to enhance the pump environment and user experience associated with use of the pump based on feedback form the environment, user, pump itself, or any combination thereof.

US 2016/0220743 A1 discloses a pump collection system including sensors that relay data, such as volume pumped, flow rate, and temperature data, to the user in real time, which is later used to calculate flow rate and volume for a particular session and then displayed on the user's smartphone.

### SUMMARY

In accordance with the principles of the present disclosure, a method and interactive system for enhancing pump performance and user experience with a pump are set forth. The method includes the steps of providing a system configured to input and store tags to various data generated during a session; and/or overlay any desired curves generated before or during use, feeding, wound drainage, or simulation, or generated under certain physiological states, to customize a pump session; and/or providing an open interface platform; and/or changing music or other parameter based on data received by the pump during a session; and/or starting or stopping any pumping parameter based on data received by the pump during the session; and/or configuring the pump to register the impact of changes made to the pump or environment on the user or pumping parameters during the session.

The interactive system comprises a pump system that includes components for sending and receiving and/or storing data, such as tags or other data inputs, inputting curve overlays captured by the system, altering music parameters for music played during the session based on system data to include volume, type of music, or other feature, receiving visual information such as facial recognition data from the pump user, processing and updating the system based on any data or data interaction received during the session with system components and or software, and registering impact data regarding the impact of changes made during the session, as well as components for correlating the data or data interaction sets to register the impact of changes made during the session and to alter the pump or environment based on the impact data in real time to improve the user experience or session efficiency.

Various advantages of the present disclosure are specifically described below in reference to the exemplary embodiments, or conceptually embodied therein. The drawings and description herein are provided to merely illustrate examples of the general concepts discussed throughout the present disclosure. Numerous changes and modifications can be made, as known to those of skill in the art, without departing from the general principles set forth herein.

### BRIEF DESCRIPTION OF THE DRAWINGS

These and other features and advantages of the various exemplary embodiments disclosed herein will be better understood with respect to the following description and drawings, in which:
Figure 1 is an exemplary system configured in accordance with the principles herein wherein any function of the pump can be changed during the session based on tags or impact data;
Figure 2 is another exemplary system configured to receive feedback from a user during a session and employ the feedback to improve the session for the user;
Figure 3 is yet another system configured to analyze and correlate impact data received during a pump session; and
Figure 4 is a flow chart for a method constructed in accordance with the principles herein; common reference numerals are used throughout the drawings and the detailed description to indicate the same elements.

### DETAILED DESCRIPTION

The detailed description set forth below in connection with the appended drawings is intended as a description of certain exemplary embodiments of various system components constructed in accordance with the principles herein. These examples are not intended to represent the only embodiments or forms that may be developed or utilized according to these principles. It is further understood that the use of relational terms such as first and second, and the like are used solely to distinguish one entity from another without necessarily requiring or implying any actual such relationship or order between such entities.

Certain aspects of some embodiments constructed in accordance with the principles herein are directed toward a method and system that improve pump sessions by monitoring the pump and environment during use examples of which are set forth in the exemplary embodiments below.

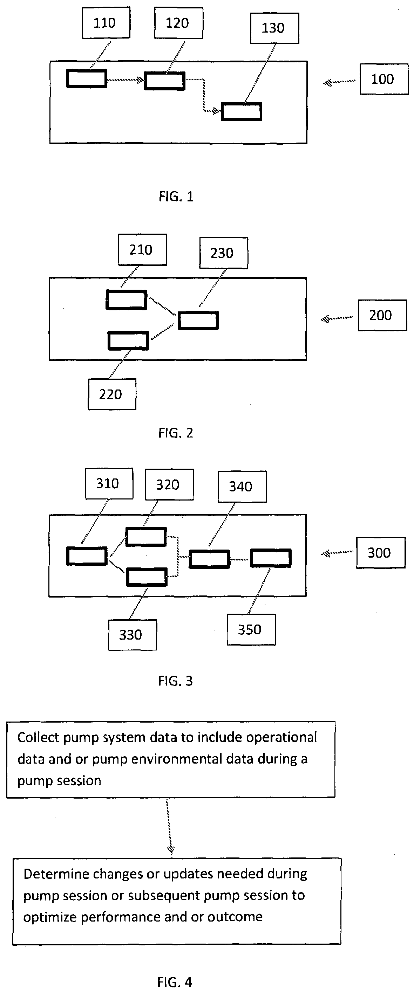

As will be described in more detail below, several embodiments are contemplated in accordance with the principles herein. For example, a first exemplary embodiment of the system shown generally at 100 is set forth in Figure 1. A second exemplary embodiment of the system shown generally at 200 is set forth in Figure 2. A third exemplary embodiment of the system shown generally at 300 is set forth in Figure 3. An exemplary method is set forth in Figure 4.

It is understood that the systems described herein may be used to collect a wide range of fluid contents from a mother or a patient. Such contents will be collectively referred to herein as "fluid" for purposes of simplicity.

An exemplary embodiment of an interactive pump system constructed in accordance with the principles herein is shown generally at 100 in Figure 1. The interactive system can be used in operating pump systems capable of correlating pump and environmental information received during a pumping session, such as a breast pump or a wound therapy pump. The system can receive data from any or all system components during the session at 110, and analyze data interactions 120 generated during the session. The system can then register impact data 130 based on data interactions 120 and analyze whether the pump operating settings or related environmental settings will update in real time based on the output of the impact data 130. The impact data can be stored directly or indirectly in a suitable memory device operably connected to the system during or after the session. For example, a user can plug in data to the pump interface. Through the open interface platform the user can use a suitable application, such as MyMedela or an enhanced version thereof consistent with the principles herein, to perform a wide variety of functions, including rating her session within the open interface. The user can time stamp logged data from one or more sensors, such as sensor data from a kit, for example. The pump open interface data can be numeric, alpha, audio visual, and can capture any data the user may want logged in real time along with pump data using open interface. In an embodiment, environmental features such as music, lighting or other environmental parameters and/or pump operation parameters, such as combining to or more sensors, can be changed or combined to form an interaction effect. For example, pumping data can be combined with facial recognition data received from a system component to alter operation of the pump in real time based on an interaction data set, determined based of the data inputs. For example, if a user has an anxious expression understood and transmitted by a facial recognition component, then the system can alter one or more parameters, such as music, lighting, or speed of the pump, to address the user's current condition. Similarly, music to stimulate flow can be initiated by the system to optimize the pump session as determined by a system component, such as a sensor and./or other components. The system can incorporate music to function as an on off switch for a pump session, or to change the way the pump is moving, or to change the system based on input from the environment and/or the user. Specifically, the system can modify any parameter, pumping or sensory stimulator, such as music, based on sensor input, user input or a research update, or any other data or signals. The user can program the pump to modify any signal in any desired way, either directly or indirectly to derive a custom pumping experience. Further, the data received by the pump and processed can pause or stop an pumping parameter, such as cycle rate; vacuum levels being achieved; shape of vacuum profile; pump intensity level or setting, and/or type of data being logged by the pump. The system can then register the impact of changes either directly or indirectly and use the data to make changes on any portion or all of a pump session about what to do next. The changes can be made based on a program and or processor operably connected to or provided within a system component, such as a breast pump, for example.

Another exemplary embodiment of an interactive pump system constructed in accordance with the principles herein is shown generally at 200 in Figure 2. The system can be configured to receive data tagged 210 sent via a user from one or more system components. The system components can include, for example, smartphones, tablets, EMR updates, research updates, system updates, user inputs to a pump interface or device operably connected to the pump system, a wireless input sending data regarding the pump system output, or any other source. More specifically, the operation parameters can be customized based on user information or other data relevant to a particular user's health, preference, or relevant research finding related to patient's use of the pump. For example, a curve for operating the pump can be determined to be preferable to certain classes of users based on research. In accordance with the principles herein, the curve can be sent to the pump interface and a picture of the curve can overlay a curve of the pump and be redrawn on the pump interface to optimize performance of the pump based on the research findings. The system can further include components 220 for tagging data generated during a pump session automatically based on stored or generated system parameters. Information that can be tagged during a session can include, for example, 1) tagging data as it is generated by the pump or by an error message inserted into the pump logs; or 2) a user giving feedback during the pumping session relating to any pumping parameter, for example the pump making noise; pain; changing the vacuum higher or lower; or the start of milk ejection reflex, which can display flags available to tag the pumping session, such as on an information screen. The system can include at least one component 230 operably connected during or after the pump session to store tagged data and or correlate tagged data and then store. If desired, the component 230 can automatically delete the tagged data from the sending component of the interactive system once received.

Yet another exemplary embodiment of an interactive system constructed in accordance with the principles herein is shown generally at 300 in Figure 3. The system includes a receiving component 310 configured to receive data and determine the status of the data. The data can be separated into data from the system components 320 and data interactions generated by the system components 330 received during the session. The system can further include one or more components 340 for determining and registering the impact of data received from the system. The system can further include components 350 that alter the pump and environment automatically, or notify the user of a recommended update based on the results of the analysis at 340 in real time during the pump session. For example, the system can start or stop any pump parameter based on data received by pump during a pump session.

As illustrated in Figure 4, an exemplary method according to the principles herein includes the steps of collecting data relevant to a pump session from at least one of expected operational parameters and environmental data; and determining needed changes during the session to optimize performance and/or outcome of the pump session for the user.

## Claims

1. An interactive pump system comprising:
at least one component configured to process and update the system during a pumping session based on data generated or data interactions generated during the pumping session, the system further configured to register impact data regarding the impact of changes made to the pump or environment on the user or pumping parameters during the pumping session in response to the data generated or data interactions generated during the pumping session, **characterized by** at least one component configured to send and receive and/or store data tags, input curve overlays captured by the system, alter music parameters for music played during the session based on system data to include volume, type of music, or other feature, receive visual information such as facial recognition data from the pump user, process and update the system based on any data or data interaction received during the session, and register impact data regarding the impact of changes made to the pump or environment on the user or pumping parameters during the session, as well as correlate the data or data interaction sets gathered during the session to register the impact of changes made to the pump or environment on the user or pumping parameters during the session and to alter the pump or environment based on the impact data in real time during the session to improve the user experience or session efficiency.

2. The interactive pump system of claim 1, the interactive pump system comprising:
a system component configured to tag and store data generated during the pumping session that is generated by the system automatically or input during the pumping session by a user.

## Patentansprüche

1. Interaktives Pumpsystem, das umfasst:
wenigstens eine Komponente, die so konfiguriert ist, dass sie Verarbeitung und Aktualisierung des Systems während einer Pumpsitzung basierend auf während der Pumpsitzung erzeugter Daten oder Dateninteraktionen durchführt,
wobei das System des Weiteren so konfiguriert ist, dass es Auswirkungsdaten registriert, die die Auswirkung an der Pumpe oder der Umgebung vorgenommener Änderungen auf den Benutzer oder Pumpparameter während der Pumpsitzung in Reaktion auf die während der Pumpsitzung erzeugten Daten oder Dateninteraktionen betreffen, **gekennzeichnet durch** wenigstens eine Komponente, die so konfiguriert ist, dass sie Daten-Tags sendet und empfängt und/oder speichert, vom System erfasste Eingabekurven-Overlays eingibt, Musikparameter für die während der Sitzung abgespielte Musik basierend auf Systemdaten, einschließlich Lautstärke, Musikrichtung oder anderer Merkmale, ändert, visuelle Informationen, wie Gesichtserkennungsdaten, von dem Nutzer der Pumpe empfängt, Verarbeitung und Aktualisierung des Systems basierend auf jeglichen Daten oder Dateninteraktionen, die während der Sitzung empfangen werden, durchführt, und Auswirkungsdaten registriert, die die Auswirkung an der Pumpe oder der Umgebung vorgenommener Änderungen auf den Benutzer oder Pumpparameter während der Sitzung betreffen, und die Sätze von Daten oder Dateninteraktionen korreliert, die während der Sitzung gesammelt werden, um die Auswirkung von Änderungen an der Pumpe oder der Umgebung vorgenommener Änderungen auf den Benutzer oder Pumpparameter während der Sitzung zu registrieren, und die Pumpe oder die Umgebung auf Basis der Auswirkungsdaten in Echtzeit während der Sitzung ändert, um die Benutzererfahrung oder die Effizienz der Sitzung zu verbessern.

2. Interaktives Pumpsystem nach Anspruch 1,
wobei das interaktive Pumpsystem umfasst:
eine Systemkomponente, die so konfiguriert ist, dass sie während der Pumpsitzung erzeugte Daten markiert und speichert, die von dem System automatisch generiert oder während der Pumpsitzung von einem Benutzer eingegeben werden.

## Revendications

1. Système de pompage interactif comprenant :
au moins un composant configuré pour traiter et mettre à jour le système durant une session de pompage sur la base de données ou d'interactions de données générées durant la session de pompage, le système étant en outre configuré pour enregistrer les données d'impact relatives à l'impact des modifications apportées à la pompe ou à l'environnement sur l'utilisateur ou les paramètres de pompage durant la session de pompage en réponse aux données ou aux interactions de données générées durant la session de pompage,
**caractérisé par** au moins un composant configuré pour envoyer, recevoir et/ou stocker des étiquettes de données, entrer des superpositions de courbes capturées par le système, modifier les paramètres musicaux de la musique diffusée durant la session sur la base des données système pour inclure le volume, le type de musique ou d'autres caractéristiques, recevoir de l'information visuelle telle que des données de reconnaissance faciale provenant de l'utilisateur de la pompe, traiter et mettre à jour le système sur la base de toutes les données ou interactions de données reçues durant la session, et enregistrer les données d'impact relatives à l'impact des modifications apportées à la pompe ou à l'environnement sur l'utilisateur ou les paramètres de pompage durant la session, ainsi que pour corréler les ensembles de données ou d'interactions de données recueillis durant la session afin d'enregistrer l'impact des modifications apportées à la pompe ou à l'environnement sur l'utilisateur ou les paramètres de pompage durant la session et de modifier la pompe ou l'environnement sur la base des données d'impact en temps réel durant la session pour améliorer l'expérience de l'utilisateur ou l'efficacité de la session.

2. Système de pompe interactif selon la revendication 1, le système de pompage interactif comprenant :
un composant système configuré pour étiqueter et stocker les données générées durant la session de pompage, qui sont générées automatiquement par le système ou saisies par un utilisateur durant la session de pompage.
